# EUROPEAN PATENT APPLICATION

(11) **EP 4 226 966 A1**
(43) Date of publication of application: **16.08.2023**
(21) Application number: 22156643.3
(22) Date of filing: 14.02.2022
(51) Int. Cl.: A61N 1/05

(54) **IMPLANTABLE LEAD AND METHOD FOR MANUFACTURING THE IMPLANTABLE LEAD**

(71) Applicant: BIOTRONIK SE & Co. KG, 12359 Berlin (DE)
(72) Inventor: Willenberg, Patrick, 03096 Dissen-Striesow (DE); Täubert, Kerstin, 12589 Berlin (DE); Müller, Tobias, 10365 Berlin (DE); Schaarschmidt, Thomas, 15732 Schulzendorf (DE); Kaiser, Dajana, 12247 Berlin (DE)
(74) Representative: Biotronik Corporate Services SE

(57) **Abstract**

The present disclosure provides an implantable lead (200, 300), comprising an elongated body (100) defining a longitudinal axis (LA) and having a circumferential enveloping plane (EP), wherein the elongated body (100) has a plurality of channels (110) extending along the longitudinal axis (LA), wherein each of the plurality of channels (110) has a channel opening (112) at the circumferential enveloping plane (EP) extending along the longitudinal axis (LA) of the elongated body (100), wherein each of the plurality of channels (110) has a circular shape in a plane perpendicular to the longitudinal axis (LA), wherein the circular shape has a radius (R), and wherein a shortest distance (SD) between a center (C) of the circular shape and the circumferential enveloping plane (EP) of the elongated body (100) is larger than zero and smaller than the radius (R); and a plurality of electrical conductors (130), wherein each of the plurality of channels (110) accommodates a respective electrical conductor of the plurality of electrical conductors (130).

## Description

Embodiments of the present disclosure relate to an implantable lead and a method for manufacturing the implantable lead. Embodiments of the present disclosure more particularly relate to semi-open lumens for installation of cable-shaped supply lines.

Medical implants are widely used to treat, replace, support and/or enhance biological structures of patients. Some medical implants are, or include, implantable leads having one or more electrodes (electrodes in this context are also denoted as electrode poles). For example, the implantable leads can be used for neurostimulation to treat chronic diseases such as chronic pain, incontinence, Parkinson, depression, and epilepsy. At least two different types of conductors can be used as supply lines to electrodes: helical leads and cable-shaped straight leads.

Due to their plastic deformability, wires can be wound into helical shaped conductors relatively easily. However, due to this plastic deformability, helical shaped conductors should have a gentle pitch, as otherwise a durability of the wires will be affected. If more than four conductors to electrodes are required, such as in spinal cord stimulation (SCS), the use of helical shaped conductors can be problematic. For example, when eight wires are wound parallel to a helix, the single wires have a high pitch, which greatly increases the stiffness of the helix and worsen the durability / fatigue performance. Furthermore, if two helixes with four individual wires each are mounted in two layers, for example, the outer diameter of the lead is increased.

If eight cable-shaped straight conductors (straight cables) are used instead of the helical shaped conductors, they can either be mounted on an inner tube in a ring-shaped single lumen or inserted into eight small satellite lumens. In a single lumen, the straight cable conductors are first mounted on the inner tube and then an outer tube is mounted. This mounting has the advantage of easy access. However, the distances between the straight cable conductors are undefined and can change over time. In addition, the straight cable conductors are not easily fixed to the lumen, which makes mounting the outer tube more difficult. If the straight cable conductors are mounted in individual small satellite lumens, care must be taken to ensure that the coefficients of friction between the lumen wall and the straight cable conductors are as low as possible and that the diameters are matched to ensure that the straight cable conductors can be inserted into the lumen. However, inserting the straight cable conductors into the lumens remains laborious and increases the risk of compression and buckling of the straight cable conductors.

In light of the above, an implantable lead and a method for manufacturing the implantable lead that overcome at least some of the problems in the art are beneficial.

It is an object of the present disclosure to provide an implantable lead and a method for manufacturing the implantable lead which can improve a structural stability and/or durability of the implantable lead. A further object of the present disclosure is to facilitate a manufacturing process of the implantable lead.

The objects are solved by the features of the independent claims. Preferred embodiments are defined in the dependent claims.

According to an independent aspect of the present disclosure, an implantable lead is provided. The implantable lead includes an elongated body defining a longitudinal axis and having a circumferential enveloping plane, wherein the elongated body has a plurality of channels extending along the longitudinal axis, wherein each of the plurality of channels has a channel opening at the circumferential enveloping plane extending along the longitudinal axis of the elongated body, wherein each of the plurality of channels has a substantial circular shape in a plane perpendicular to the longitudinal axis, wherein the circular shape has a radius, and wherein a shortest distance between a center of the circular shape and the circumferential enveloping plane of the elongated body is larger than zero and smaller than the radius; and a plurality of electrical conductors, wherein each of the plurality of channels accommodates a respective electrical conductor of the plurality of electrical conductors.

According to some embodiments, which can be combined with other embodiments described herein, the implantable lead further includes a plurality of electrodes connected to the plurality of electrical conductors. The plurality of electrodes may provide a therapeutic function, such as neurostimulation, when implanted inside a patient's body.

Preferably, each channel of the plurality of channels has an Ω (Omega)-shape in a plane substantially perpendicular to the longitudinal axis of the elongated body.

Preferably, the plurality of channels has a substantial circular shape in a plane perpendicular to the longitudinal axis, whereas the channels may also have an oval shape.

According to some embodiments, which can be combined with other embodiments described herein, a width of the channel opening is at least 10%, preferably at least 25%, and more preferably at least 50% smaller than a diameter of the electrical conductor in the respective channel. The width of the channel opening can be defined in a direction substantially perpendicular to the longitudinal axis of the elongated body.

According to some embodiments, which can be combined with other embodiments described herein, the radius of the circular shape is 0.5 mm or less, preferably 0.3 mm or less, and more preferably 0.1 mm or less. Accordingly, the diameter (=2·R) of the circular shape can be 1 mm or less, preferably 0.6 mm or less, and more preferably 0.2 mm or less.

According to some embodiments, which can be combined with other embodiments described herein, the plurality of channels are four or more channels, or eighth or more channels.

According to some embodiments, which can be combined with other embodiments described herein, the plurality of channels may be provided on different radiuses of the elongated body, e.g. one radius might be closer to the enveloping plane.

According to some embodiments, which can be combined with other embodiments described herein, the plurality of channels may have different sizes or radiuses. Thus, there might be small and larger channels distributed around the elongated body.

According to some embodiments, which can be combined with other embodiments described herein, the plurality of channels are circumferentially spaced apart from one another around the longitudinal axis of the elongated body. In particular, the plurality of channels can be equidistantly spaced apart from each other in a circumference direction of the elongated body.

According to some embodiments, which can be combined with other embodiments described herein, the elongated body includes a center bore extending along the longitudinal axis. The center bore can also be referred to as "central lumen" or "inner lumen".

Preferably, a diameter of the center bore is 1 mm or less, more preferably 0.5 mm or less.

According to some embodiments, which can be combined with other embodiments described herein, the elongated body is formed of an insulating material. Preferably, the insulating material includes, or is, polyurethane and/or silicone. However, the present disclosure is not limited thereto and other single insulating materials or combinations of insulating materials can be used for the elongated body.

According to some embodiments, which can be combined with other embodiments described herein, the implantable lead further includes a sleeve. Preferably, the elongated body having the plurality of electrical conductors mounted thereon is located inside the sleeve.

Preferably, the sleeve is formed of an insulating material. In some embodiments, the insulating material includes, or is, polyurethane and/or silicone. However, the present disclosure is not limited thereto and other single insulating materials or combinations of insulating materials can be used for the sleeve.

According to some embodiments, which can be combined with other embodiments described herein, the elongated body and the sleeve are formed or made of the same insulating material. In other embodiments, the elongated body and the sleeve are formed or made of different insulating materials.

According to some embodiments, which can be combined with other embodiments described herein, the implantable lead further includes at least one wire or cable wound around the elongated body. Preferably, the at least one wire or cable is located between the elongated body and the sleeve. The wire or cable may not fulfill any therapeutic function but lead to a reduction of RF-induced heating at the plurality of electrodes when using, for example, a magnetic resonance tomograph.

According to another independent aspect of the present disclosure, a medical implant is provided. The medical implant is, or includes, one or more implantable leads of the embodiments described herein.

According to some embodiments, which can be combined with other embodiments described herein, the medical implant is configured for neurostimulation. However, the present disclosure is not limited thereto, and the medical implant, particularly the implantable lead, may also be used in other medical applications, such as catheters or CRM electrodes.

According to another independent aspect of the present disclosure, a method of manufacturing an implantable lead is provided. The method includes forming an elongated body, wherein the elongated body defines a longitudinal axis and a circumferential enveloping plane, wherein the elongated body has a plurality of channels extending along the longitudinal axis, wherein each of the plurality of channels has a channel opening at the circumferential enveloping plane extending along the longitudinal axis of the elongated body, wherein each of the plurality of channels has a circular shape in a plane perpendicular to the longitudinal axis, wherein the circular shape has a radius, and wherein a shortest distance between a center of the circular shape and the circumferential enveloping plane of the elongated body is larger than zero and smaller than the radius; and inserting a plurality of electrical conductors in the elongated body such that each of the plurality of channels accommodates a respective electrical conductor of the plurality of electrical conductors.

Preferably, the elongated body is formed by an extrusion process, such as plastic extrusion.

So that the manner in which the above recited features of the present disclosure can be understood in detail, a more particular description of the disclosure, briefly summarized above, may be had by reference to embodiments. The accompanying drawings relate to embodiments of the disclosure and are described in the following:
- FIG. 1A: shows a perspective view of an elongated body of an implantable lead according to embodiments of the present disclosure;
- FIGs. 1B-D: show cross-sectional views of the elongated body of FIG. 1A;
- FIGs. 2A-C: show a manufacturing process of an implantable lead of a first embodiment of the present disclosure; and
- FIGs. 3A-D: show a manufacturing process of an implantable lead of a second embodiment of the present disclosure.

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure.

Further, features illustrated or described as part of one embodiment can be used on or in conjunction with other embodiments to yield yet a further embodiment. It is intended that the description includes such modifications and variations.

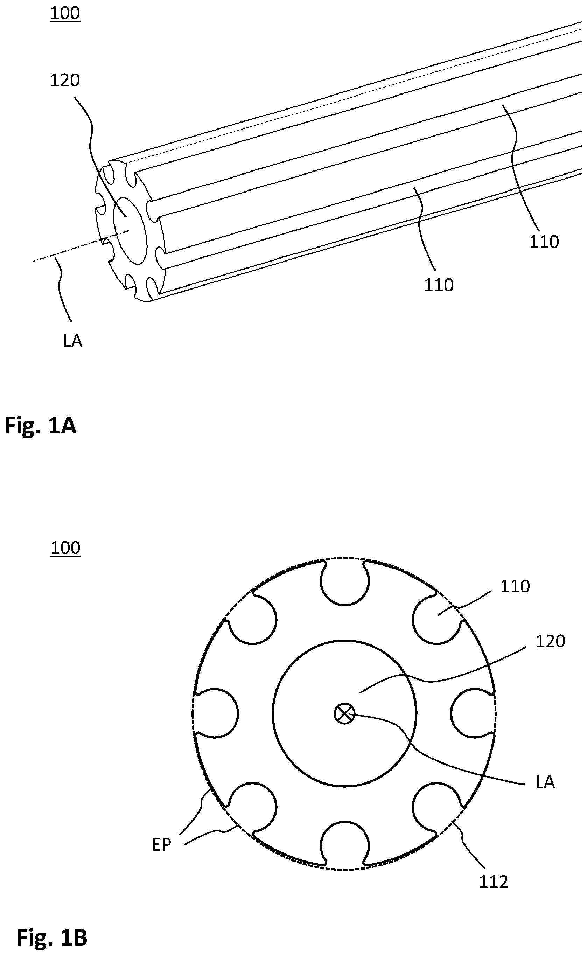

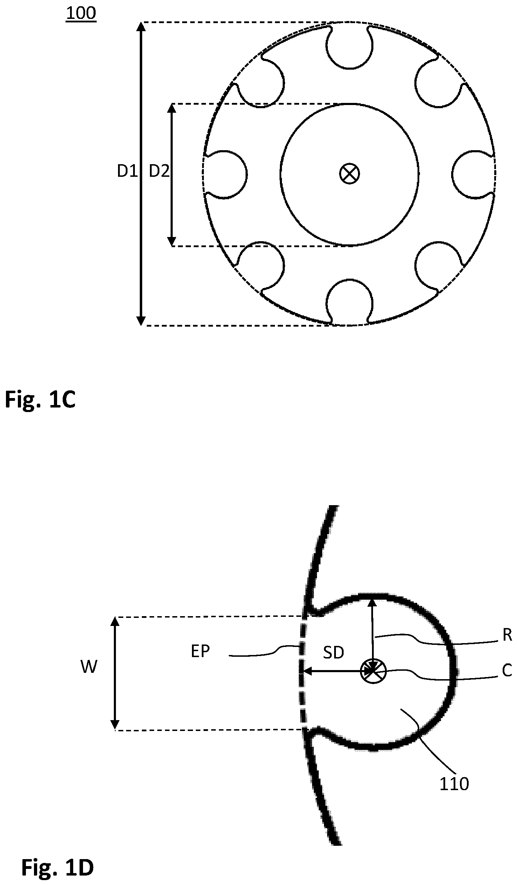

FIG. 1A shows a perspective view of an elongated body 100 of an implantable lead according to embodiments of the present disclosure. FIGs. 1B-D show cross-sectional views of the elongated body 100 of FIG. 1A.

The elongated body 100 of the implantable lead may be formed of an insulating material. Preferably, the insulating material includes, or is, polyurethane and/or silicone. In some embodiments, the elongated body 100 can be manufactured by extrusion, such as plastic extrusion.

The elongated body 100 has or defines a longitudinal axis LA. In some embodiments, the longitudinal axis LA can be a center axis of the elongated body 100. The elongated body 100 can have an outer diameter D1 of 2 mm or less, preferably 1.5 mm or less, and more preferably 1 mm or less.

In some embodiments, the elongated body 100 may include a center bore 120 extending along the longitudinal axis LA. The center bore 120 can also be referred to as "central lumen" or "inner lumen". The center bore 120 can have a diameter D2 of 1 mm or less, preferably 0.5 mm or less. In an exemplary and non-limiting embodiment, the elongated body 100 can have an outer diameter D1 of 0.95 mm and the center bore 120 can have a diameter D2 of 0.45 mm.

The elongated body 100 includes a plurality of channels 110 extending along the longitudinal axis LA. The plurality of channels 110 are formed in an outer surface of the elongated body 100. In particular, the plurality of channels 110 are open channels provided as some sort of recess or groove on the outer surface of the elongated body 100.

Each channel of the plurality of channels 110 can be configured to accommodate an electrical conductor (not shown). Furthermore, each channel of the plurality of channels 110 has a channel opening 112 that can be used to insert the electrical conductor into the channel. In some embodiments, the plurality of channels 110 are four or more channels, or eighth or more channels.

The plurality of channels 110 can be circumferentially spaced apart from one another around the longitudinal axis LA of the elongated body 100. In particular, the plurality of channels 110 can be equidistantly spaced apart from each other in a circumference direction of the elongated body 100. The circumference direction can be substantially perpendicular to the longitudinal axis LA of the elongated body 100.

The elongated body 100 has or defines a circumferential enveloping plane EP, which encloses the elongated body 100. In particular, the circumferential enveloping plane EP is a plane which conforms with the outer surface of the elongated body 100 and covers the channel openings 112 of the plurality of channels 110. Accordingly, the channel openings 112 are provided at the circumferential enveloping plane EP.

In some embodiments, the circumferential enveloping plane EP has an essentially cylindrical shape. A cylinder axis of the cylindrical shape and the longitudinal axis LA of the elongated body 100 may coincide.

Preferably, each channel of the plurality of channels 110 has an Ω (Omega)-shape in a plane substantially perpendicular to the longitudinal axis LA of the elongated body 100. The channel opening 112 can correspond to the opening at the bottom of the Ω (Omega)-shape. A portion of the channel opening 112 corresponding to the top of the Ω (Omega)-shape can face towards a center of the elongated body 100, and in particular to the longitudinal axis LA.

Each of the plurality of channels 110 has a circular shape in a plane perpendicular to the longitudinal axis LA. The circular shape is defined by the inner surface of the channel 110 and has a radius R. A shortest distance SD a between a center C of the circular shape and the circumferential enveloping plane EP of the elongated body 100 is larger than zero and smaller than the radius R of the circular shape. Consequently, a width W of the channel opening 112 in a direction substantially perpendicular to the longitudinal axis LA of the elongated body 100 is less than a diameter (=2·R) of the circular shape. Therefore, the channel has the Ω (Omega)-shape in the plane substantially perpendicular to the longitudinal axis LA of the elongated body 100. In other words the cannel opening 112 is smaller as the diameter of the channel 110. The channel 110 might also has a C-form.

This geometry of the channels 110 allows easy access and the electrical conductors can be clipped into the channels 110 from the outside. This fixes the electrical conductors to the elongated body 100 at fixed distances from each other. This can simplify the assembly of, for example, straight cable conductors, while at the same time radial fixation of the straight cable conductors can be achieved.

According to some embodiments, the width W of the channel opening 112 is at least 10%, preferably at least 25%, and more preferably at least 50% smaller than a diameter of the electrical conductor in the respective channel. This can ensure that the electrical conductor is fixed in the channel.

Preferably, the diameter of the electrical conductor can be 0.3 mm or less, preferably 0.2 mm or less, and more preferably 0.15 mm or less. In an exemplary and non-limiting embodiment, the diameter of the electrical conductor can be 0.145 mm and the width W of the channel opening 112 can be 0.12 mm or 0.13 mm.

Preferably, the radius R of the circular shape is 0.5 mm or less, preferably 0.3 mm or less, and more preferably 0.1 mm or less. Accordingly, the diameter of the circular shape can be 1 mm or less, preferably 0.6 mm or less, and more preferably 0.2 mm or less.

In some embodiments, the diameter of the circular shape can be essentially equal to, or less than, the diameter of the electrical conductor.

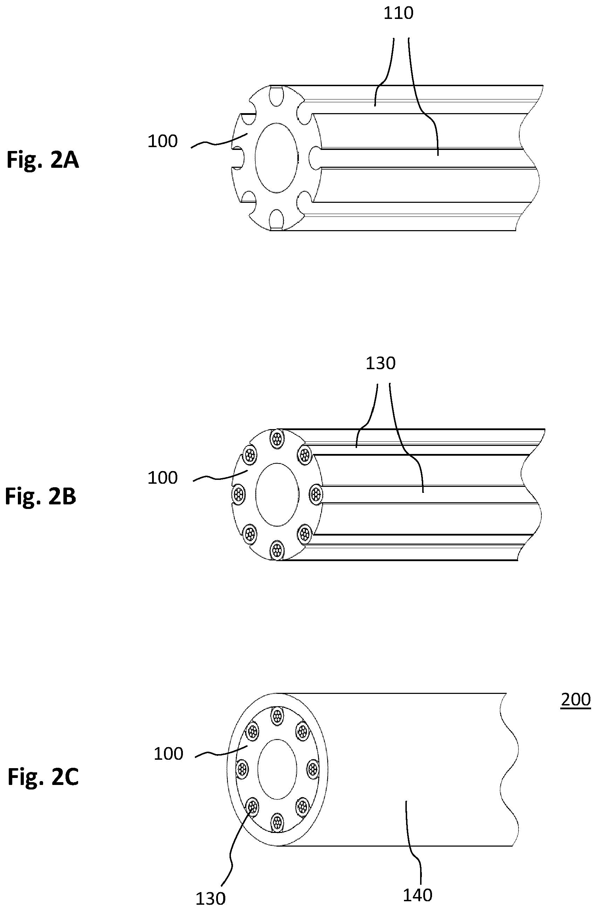

FIGs. 2A-C show a manufacturing process of an implantable lead 200 of a first embodiment of the present disclosure.

FIG. 2A shows the elongated body 100 which has been described with respect to FIGs. 1A- D. The elongated body 100 can be manufactured by extrusion, such as plastic extrusion.

As shown in FIG. 2B, in each of the channels 110 a respective electrical conductor 130 is inserted or clipped. The electrical conductor 130 can be a straight cable conductor or wire. For example, one straight cable conductor (e.g., DFT conductor, MP35N 28% Ag and ETFE insulation) with a diameter of, for example, 0.145 mm can be inserted into each of the channels 110 with slight pressure. When the assembly is pulled through a sleeve-shaped orifice, all electrical conductors 130 are simultaneously pressed into the respective channels 110 over the entire length of the elongated body 100 and, due to the Ω-shape of the channels, radially fixed in the channels 110 for further assembly steps.

In some embodiments, as shown in FIG. 2C, a sleeve 140 can be provided in which the elongated body 100 having the plurality of electrical conductors 130 mounted thereon can be inserted. For example, after mounting all electrical conductors 130, an outer sleeve with an inner diameter of, for example, 1.13 mm and an outer diameter of, for example, 1.33 mm can be mounted on the elongated body 100 having the plurality of electrical conductors 130 mounted thereon.

Preferably, the sleeve 140 is formed of an insulating material. In some embodiments, the insulating material includes, or is, polyurethane and/or silicone. According to some embodiments, the elongated body 100 and the sleeve 140 can be formed or made of the same insulating material. In other embodiments, the elongated body 100 and the sleeve 140 can be formed or made of different insulating materials.

In some embodiments, the implantable lead 200 further includes a plurality of electrodes (not shown) connected to the plurality of electrical conductors 140. The plurality of electrodes may provide a therapeutic function, such as neurostimulation, when implanted inside a patient's body. However, the present disclosure is not limited thereto, and the implantable lead 200 may also be used in other medical applications, such as catheters or CRM electrodes.

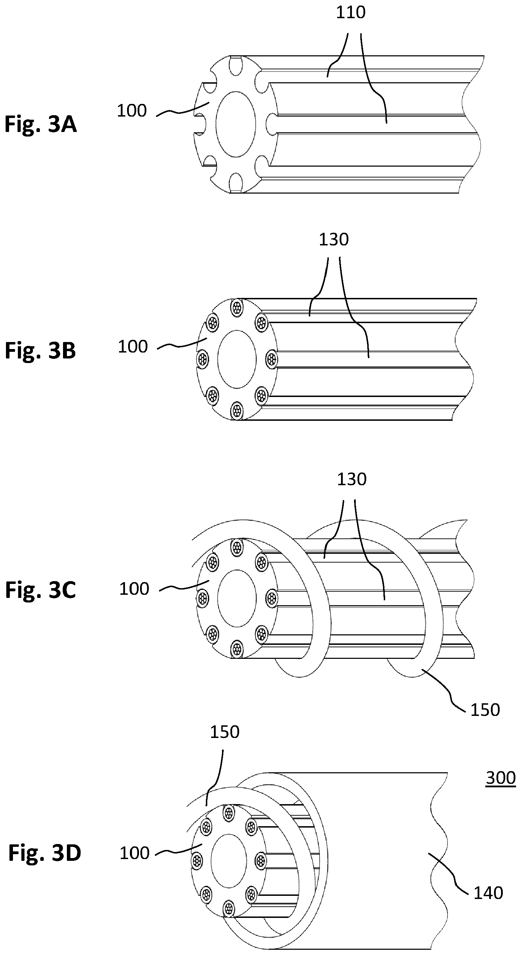

FIGs. 3A-D show a manufacturing process of an implantable lead 300 of a second embodiment of the present disclosure.

The second embodiment is similar to the first embodiment and therefore, the description of similar or identical aspects and features will not be repeated.

The implantable lead 300 of the second embodiment further includes at least one wire or cable 150 wound around the elongated body 100. Preferably, the at least one wire or cable 150 is located between the elongated body 100 and the sleeve 140. The at least one wire or cable 150 may have a diameter of 0.5 mm or less, preferably 0.3 mm or less, and more preferably 0.1 mm or less. In an exemplary and non-limiting embodiment, the at least one wire or cable 150 may have a diameter of about 0.145 mm.

The wire or cable 150 may not fulfill any therapeutic function but lead to a reduction of RF-induced heating at the plurality of electrodes when using a magnetic resonance tomograph. In this case, the open channels can have the additional advantage that the therapeutic conductors 130 and the wire or cable 150 couple more capacitively with each other due to the reduced insulation between them, and the reducing effect on RF heating is enhanced. Another positive effect of this close arrangement of therapeutic conductors 130 and wire or cable 150 is the reduction in the overall outer diameter of the implantable lead compared to a multi-lumen tube with closed satellite lumens.

After mounting the electrical conductors 130 and the wire or cable 150, the outer sleeve 140 can be mounted. The sleeve 140 can have an inner diameter of, for example, 1.26 mm or 1.28 mm and an outer diameter of, for example, 1.46 mm or 1.48 mm.

The implantable lead of the embodiments of the present disclosure provides at least some of the following beneficial effects:
- Simple, simultaneous assembly of several cable-shaped therapeutic conductors: time savings in the manufacturing process and/or reduction of employee influence and scrap rate due to the avoidance of manual threading of the therapeutic conductors through closed satellite lumens and/or radial fixation of the therapeutic conductors for further assembly steps without additional tools, devices or processes (e.g., gluing).
- Defined distance between the therapeutic conductors.
- Proximity of therapeutic conductors and helical wire or cable , increasing the reducing effect on RF heating.
- Reduction of the total diameter of the implantable lead.

While the foregoing is directed to embodiments of the disclosure, other and further embodiments of the disclosure may be devised without departing from the basic scope thereof, and the scope thereof is determined by the claims that follow.

## Claims

1. An implantable lead (200, 300), comprising:
- an elongated body (100) defining a longitudinal axis (LA) and having a circumferential enveloping plane (EP), wherein the elongated body (100) has a plurality of channels (110) extending along the longitudinal axis (LA), wherein each of the plurality of channels (110) has a channel opening (112) at the circumferential enveloping plane (EP) extending along the longitudinal axis (LA) of the elongated body (100), wherein each of the plurality of channels (110) has a substantial circular shape in a plane perpendicular to the longitudinal axis (LA), wherein the circular shape has a radius (R), and wherein a shortest distance (SD) between a center (C) of the circular shape and the circumferential enveloping plane (EP) of the elongated body (100) is larger than zero and smaller than the radius (R); and
- a plurality of electrical conductors (130), wherein each of the plurality of channels (110) accommodates a respective electrical conductor of the plurality of electrical conductors (130).

2. The implantable lead (200, 300) of claim 1, wherein a width (W) of the channel opening (112) is at least 10%, preferably at least 25%, and more preferably at least 50% smaller than a diameter of the electrical conductor (130) in the respective channel (110).

3. The implantable lead (200, 300) of claim 1 or 2, wherein the radius (R) of the circular shape is 0.5 mm or less, preferably 0.3 mm or less, and more preferably 0.1 mm or less.

4. The implantable lead (200, 300) of any one of claims 1 to 3, wherein the plurality of channels (110) are four or more channels, or eighth or more channels.

5. The implantable lead (200, 300) of any one of claims 1 to 4, wherein the plurality of channels (110) are equidistantly spaced apart from each other in a circumference direction of the elongated body (100).

6. The implantable lead (200, 300) of any one of claims 1 to 5, wherein the elongated body (100) includes a center bore (120) extending along the longitudinal axis (LA).

7. The implantable lead (200, 300) of claim 6, wherein a diameter (D2) of the center bore (120) is 1 mm or less, preferably 0.5 mm or less.

8. The implantable lead (200, 300) of any one of claims 1 to 7, wherein the elongated body (100) is formed of an insulating material, in particular polyurethane and/or silicone.

9. The implantable lead (200, 300) of any one of claims 1 to 8, further including a sleeve (140), wherein the elongated body (100) having the plurality of electrical conductors (130) mounted thereon is located inside the sleeve (140).

10. The implantable lead (200, 300) of claim 9, wherein the sleeve (140) is formed of an insulating material, in particular polyurethane and/or silicone.

11. The implantable lead (300) of any one of claims 1 to 10, further including at least one wire or cable conductor (150) wound around the elongated body (100), in particular wherein the at least one wire or cable conductor (150) is located between the elongated body (100) and the sleeve (140).

12. The implantable lead (200, 300) of any one of claims 1 to 11, further including a plurality of electrodes connected to the plurality of electrical conductors (130).

13. A medical implant, including one or more implantable leads (200, 300) of any one of claims 1 to 12.

14. The medical implant of claim 13, wherein the medical implant is configured for neurostimulation.

15. A method of manufacturing an implantable lead, comprising:
- forming an elongated body, wherein the elongated body defines a longitudinal axis and a circumferential enveloping plane, wherein the elongated body has a plurality of channels extending along the longitudinal axis, wherein each of the plurality of channels has a channel opening at the circumferential enveloping plane extending along the longitudinal axis of the elongated body, wherein each of the plurality of channels has a circular shape in a plane perpendicular to the longitudinal axis, wherein the circular shape has a radius, and wherein a shortest distance between a center of the circular shape and the circumferential enveloping plane of the elongated body is larger than zero and smaller than the radius; and
- inserting a plurality of electrical conductors in the elongated body such that each of the plurality of channels accommodates a respective electrical conductor of the plurality of electrical conductors.
